# EUROPÄISCHE PATENTANMELDUNG

(11) **EP 1 611 905 A1**
(43) Veröffentlichungstag der Anmeldung: **04.01.2006**
(21) Anmeldenummer: 04103008.1
(22) Anmeldetag: 28.06.2004
(51) Int. Cl.: A61L 9/14, B05B 17/06, F24F 3/12, F24F 3/16, A61L 9/04

(54) **Gerät zum Einbringen eines flüssigen Mediums, insbesondere Entkeimungsmittels in ein gasförmiges Medium**

(71) Anmelder: Anti-Germ AG, 3063 Ittigen (CH)
(72) Erfinder: Bolt, Ernst, 3048, Worblaufen (CH); Strahm, Peter, 3065, Bolligen (CH); Suter, Hansruedi, 3065, Bolligen (CH)
(74) Vertreter: BOVARD AG

(57) **Zusammenfassung**

Ein Gerät zum Einbringen eines Entkeimungsmittels (16) in ein gasförmiges Medium umfasst einen Gehäuseteil (1), in welchem eine Vernebelungseinrichtung (2) in Form eines Ultraschall-Zerstäubers mit einem Piezoelement (3) untergebracht ist. Das in flüssiger Form vorliegende, in einem Behälter enthaltene Entkeimungsmittel (16) ist dem Piezoelement zuführbar, und wird zerstäubt. Über einen Kanal wird ein gasförmiges Medium am Ultraschall-Zerstäuber vorbeigeführt und nimmt die zerstäubten Partikel des Entkeimungsmittels (16) mit. Im Kanal ist ein Ventilator (7) angeordnet, zusätzlich ist im Gerät eine Stromversorgungseinheit für den Ultraschall-Zerstäuber und den Ventilator und für eine Steuereinheit (14) untergebracht. Über die Steuereinheit (14) ist das Piezoelement (3) des Ultraschall-Zerstäubers mit einer vorgegebenen Frequenz, die im Bereich der Eigenfrequenz des Piezoelementes (3) liegt, in Schwingung versetzbar. Über eine zusätzlich angebrachte Spannungssteuereinheit (17) kann die an den Ultraschall-Zerstäuber angelegte Spannung verändert werden. Dadurch lässt sich, zusammen mit der Regelung des Ventilators, eine einfache und optimale Regelung der zerstäubten Menge des Entkeimungsmittels erreichen.

## Beschreibung

Die vorliegende Erfindung bezieht sich auf ein Gerät zum Einbringen eines flüssigen Mediums, insbesondere Entkeimungsmittels in ein gasförmiges Medium, umfassend einen Gehäuseteil, in welchem eine Vernebelungseinrichtung in Form eines Ultraschall-Zerstäubers mit einem Piezoelement, welchem das in flüssiger Form vorliegende, in einem Behälter enthaltene Entkeimungsmittel zur Zerstäubung zuführbar ist, ein Kanal mit einer Einlassöffnung und mindestens einer Auslassöffnung, durch welchen das gasförmige Medium am Ultraschall-Zerstäuber vorbeiführbar ist und die zerstäubten Partikel des Entkeimungsmittels mitnimmt, ein im Kanal angeordneter Ventilator, eine Stromversorgungseinheit für den Ultraschall-Zerstäuber und den Ventilator und eine Steuereinheit untergebracht sind.

Es ist bekannt, dass in Klimaanlangen, Lüftungseinrichtungen und Luftumwälzungen in Gebäudeanlagen und Räumlichkeiten mit der Luftströmung Keime mitgerissen werden, die vielfältiger Art sind, beispielsweise Pilzsporen, Pilze, Bakterien, Viren usw.. Derartige Keime sind selbstverständlich unerwünscht.

Zum Bekämpfen dieser Keime sind vielfältige Lösungen bekannt. Eine bevorzugte Lösung ist beispielsweise in der EP-A-1 224 948 beschrieben, bei welcher eine Desinfektionsflüssigkeit in einem Gerät durch Anlegen hochfrequenter Wechselspannung vernebelt wird. Neben der Einbringung des Entkeimungsmittels wird hierbei auch eine Befeuchtung des gasförmigen Mediums erzielt. Die Regelung der Vernebelung wird durch die Veränderung der Frequenz des Ultraschall-Schwingers erreicht, was sich auch auf die Tröpfchengrösse auswirkt, die durch den Ultraschall-Schwinger erzeugt werden. Wenn die Tröpfchengrösse zu gross ist, kann keine optimale Verteilung des Desinfektionsmittels im entsprechenden gasförmigen Medium erreicht werden, die Wirkung wird beeinträchtigt.

Die Aufgabe der vorliegenden Erfindung besteht darin, ein Gerät zu schaffen, in welchem ein optimaler Zerstäubungsvorgang abläuft, das einfach aufgebaut ist und mit welchem die Regelung der Menge des zerstäubten flüssigen Mediums, die erzeugt wird, in einfacher Weise vorgenommen werden kann.

Zudem soll das Gerät vielfältig einsetzbar sein. Es soll auch problemlos in bereits bestehende Anlagen eingesetzt werden können.

Erfindungsgemäss erfolgt die Lösung dieser Aufgabe dadurch, dass über die Steuereinheit das Piezoelement des Ultraschall-Zerstäubers mit einer vorgegebenen Frequenz, die im Bereich der Eigenfrequenz des Piezoelementes liegt, in Schwingung versetzbar ist, und dass eine Spannungssteuereinheit angebracht ist, mit welchen die an den Ultraschall-Zerstäuber angelegte Spannung veränderbar ist.

Durch die vorgegebene Frequenz, die im Bereich der Eigenfrequenz des Piezoelementes liegt, wird eine effiziente Zerstäubung des Entkeimungsmittels erreicht. Die Grösse der erzeugten Tröpfchen bewegt sich innerhalb eines Bereichs, der gleich bleibend ist. Durch die Veränderung der Schwingungsamplitude kann die Menge der erzeugten Tröpfchen mit einer gewünschten Grösse in einfacher Weise geregelt werden, mit einer vorgegebenen Frequenz, bei welcher die Effizienz möglichst gross ist. Die Einstellung der Menge ist auch durch eine Pulsweitenmodulation der angelegten Spannung erreichbar.

In vorteilhafter Weise ist die Drehzahl des im Kanal angeordneten Ventilators über die Steuereinheit regelbar. Dadurch lässt sich die Menge der Tröpfchen, die durch das gasförmige Medium mitgenommen werden, regeln.

Eine weitere vorteilhafte Ausgestaltung der Erfindung besteht darin, dass die Stromversorgungseinheit als Modul ausgebildet ist und in das Gerät einsetzbar ist. Dadurch kann die Stromversorgungseinheit beispielsweise ein Netzgerät sein, das an das örtliche Stromnetz anschliessbar ist, die Stromversorgungseinheit kann aber auch eine Batterie sein, wodurch das Gerät netzunabhängig betrieben werden kann, was die Flexibilität des Einsatzes erhöht.

Der Behälter für die Aufnahme des flüssigen Mediums, insbesondere Entkeimungsmittels kann auf das Gerät aufsteckbar sein, wozu das Gerät mit entsprechenden Steckverbindungen ausgestattet ist. Dadurch wird die örtliche Unabhängigkeit des Einsatzes des Gerätes hinsichtlich des Behälters optimal.

Der Behälter kann auch vom Gerät getrennt aufstellbar sein, wozu dieser mit einer Leitung versehen ist, welche mit einer entsprechenden, am Gerät angebrachten Steckverbindung kuppelbar ist. Dadurch kann das Gerät langzeitig eingesetzt werden, der Behälter kann so ausgestaltet sein, dass er eine grosse Menge von flüssigem Medium aufnehmen kann, er kann auch so aufgestellt werden, dass die Befüllung des Behälters mit flüssigem Medium oder das Ersetzen eines leeren Behälters mit einem vollen Behälter optimal möglich wird.

Eine weitere vorteilhafte Ausgestaltung der Erfindung besteht darin, dass auf die Steckverbindung am Gerät ein Pumpenmodul aufsteckbar ist, das über die Stromversorgungseinrichtung speisbar ist, an welches Pumpenmodul die mit dem Behälter verbundene Leitung anschliessbar ist. Der Behälter kann somit auch unterhalb des Gerätes angeordnet sein, das Zuführen von flüssigem Medium aus dem Behälter wird durch das Pumpenmodul oder eine Zentralpumpe für mehrere Geräte gewährleistet.

Eine weitere vorteilhafte Ausgestaltung der Erfindung besteht darin, dass die mindestens eine Auslassöffnung des Kanals auf der Unterdruckseite eines Flügelprofiles angeordnet ist, welches Flügelprofil in einem Lüftungskanal einer Lüftungseinrichtung untergebracht ist. Dadurch wird das mit dem zerstäubten flüssigen Medium versehene gasförmige Medium, das durch den Kanal fliesst, durch die Saugwirkung aus dem Kanal gesaugt und in die Luft im Lüftungskanal eingebracht und in optimaler Weise verteilt, ohne dass weitere Mittel erforderlich sind.

In vorteilhafter Weise erstreckt sich das Flügelprofil über mindestens einen Teil der Breite des Lüftungskanals, und ist so befestigt, dass der Anstellwinkel des Flügelprofils zur Strömungsrichtung des im Lüftungskanals strömenden Mediums veränderbar ist. Dadurch lässt sich die Saugwirkung und demzufolge die in das strömende Medium einbringbare Menge des flüssigen Mediums einstellen.

In vorteilhafter Weise sind mehrere Auslassöffnungen über die Länge des Flügelprofils verteilt angeordnet. Dadurch lässt sich eine optimale Verteilung des flüssigen Mediums in dem im Luftkanal strömenden Medium erreichen.

Ausführungsformen der Erfindung werden nachfolgend anhand der beiliegenden Zeichnung beispielhaft näher erläutert.

Es zeigt:
Fig. 1 in räumlicher und schematischer Darstellung den modularen Aufbau des erfindungsgemässen Gerätes;
Fig. 2 eine schematische Darstellung im Schnitt des erfindungsgemässen Gerätes nach Fig. 1;
Fig. 3 eine Ansicht auf ein in einem Lüftungskanal angeordnetes Flügelprofil;
Fig. 4 in schematischer Darstellung den Einsatz eines erfindungsgemässen Gerätes in der Lüftung eines Busses;
Fig. 5 und 6 in schematischer Darstellung den Einsatz eines erfindungsgemässen Gerätes in einer Kühleinrichtung eines Kühlraumes;
Fig. 7 in schematischer Darstellung den Einsatz eines erfindungsgemässen Gerätes in der Belüftungseinrichtung eines Raumes; und
Fig. 8 in schematischer Darstellung den Einsatz eines erfindungsgemässen Gerätes in einer Einrichtung zur Bekämpfung von Fusspilz.

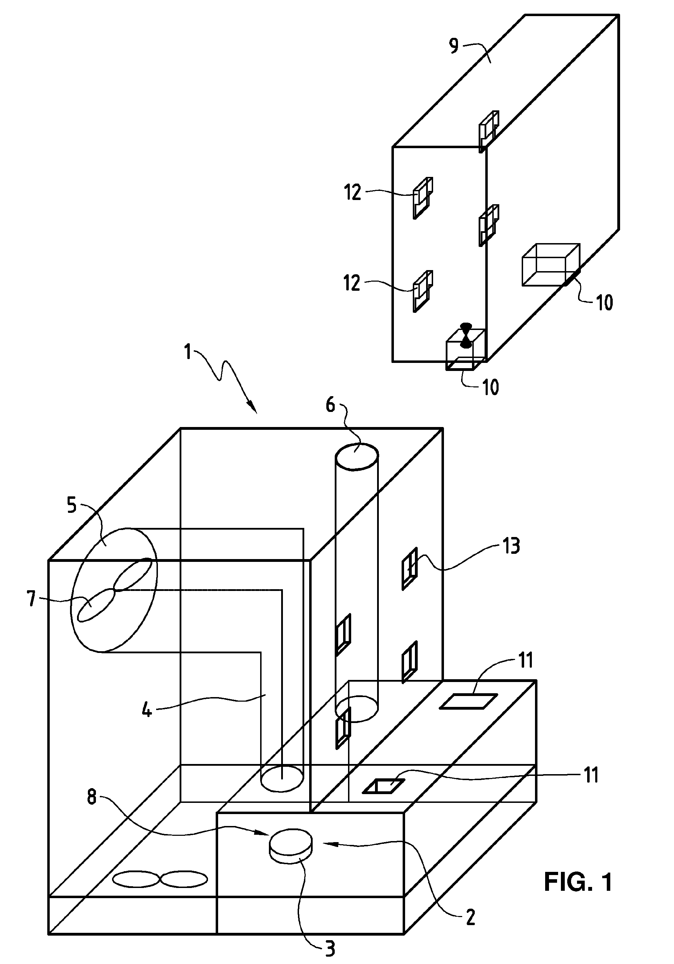

Wie aus Fig. 1 ersichtlich ist, umfasst das erfindungsgemässe Gerät, das z.B. zur Entkeimung eingesetzt wird, einen Gehäuseteil 1, in welchem die Vernebelungseinrichtung 2 in Form eines Ultraschall-Zerstäubers mit einem Piezoelement 3 untergebracht ist. Im Gehäuseteil 1 ist ein Kanal 4 eingesetzt, der eine Einlassöffnung 5 und eine Auslassöffnung 6 aufweist. Der Kanal 4 führt die durch die Einlassöffnung 5 angesaugte Luft durch den Raum 8, in welchem das Piezoelement 3 untergebracht ist, und leitet diese durch die Auslassöffnung 6 ab, deren Ausgestaltung später noch beschrieben wird.

Zur Durchströmung von Luft durch diesen Kanal 4 ist in diesem ein Ventilator 7 vorgesehen, der antreibbar ist.

Das Piezoelement 3, das sich im Raum 8 befindet, wird vom zu zerstäubenden Entkeimungsmittel überspült, das Piezoelement wird in Schwingung versetzt, beispielsweise mit einer Frequenz von etwa 1,6 MHz, abhängig von der Eigenfrequenz des Piezoelementes was in bekannter Weise zum Zerstäubungseffekt des entsprechenden Mittels führt. Über die durch den Kanal zugeführte Luft wird der so gebildete Nebel mitgenommen und durch die Auslassöffnung 6 abgegeben.

Das Entkeimungsmittel befindet sich in einem wegnehmbaren Behälter 9, der mit ersten Steckelementen 10 ausgestattet ist, mit welchen er auf zweite Steckelemente aufsteckbar ist, die am Gehäuseteil 1 des erfindungsgemässen Gerätes angebracht sind. Zusätzlich ist der Behälter noch mit Haken 12 ausgestattet, die in entsprechende Aufnahmen 13 am Gehäuseteil 1 einhängbar sind.

Das eine erste Steckelement 10 dient beispielsweise der Zuführung über das entsprechende zweite Steckelement 11 der Entkeimungsflüssigkeit in den Raum 8, während das andere erste Steckelement 10 mit dem entsprechenden zweiten Steckelement 11 eine elektrische Verbindung zwischen dem Gerät und dem Behälter 9 bilden kann.

Der Behälter 9 kann als wieder befüllbarer Behälter ausgestaltet sein, es ist auch denkbar, dass der Behälter 9 nach der Entleerung durch einen neuen, gefüllten Behälter ersetzt wird, was vom Anwendungszweck und entsprechend vom verwendeten Mittel abhängig ist.

Anstelle des Behälters 9 kann ein Pumpenmodul über die zweiten Steckelemente 11 mit dem Gehäuseteil 1 des erfindungsgemässen Gerätes verbunden werden. Hierbei kann dieses Pumpenmodul aus einem separaten Behälter, der über eine Leitung mit dem Pumpenmodul verbunden ist, das zu verarbeitende Mittel dem Gerät zuführen.

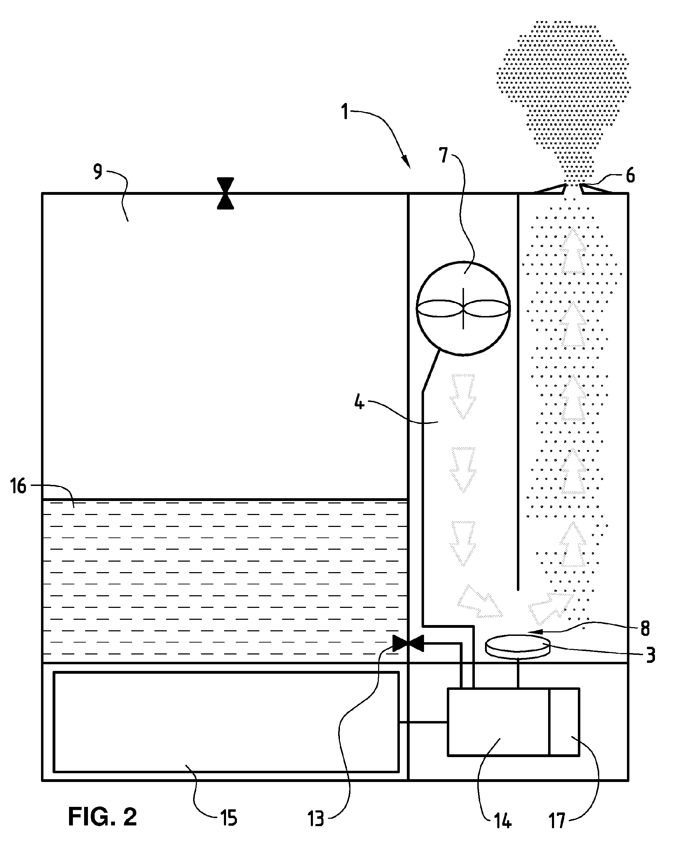

In der schematischen Darstellung gemäss Fig. 2, das ein batteriebetriebenes Kleinstgerät zeigt (beispielsweise in der Grösse einer Zigarettenpackung), ist der Behälter 9 ersichtlich, in welchem sich das Entkeimungsmittel 16 befindet. An diesem Behälter 9 ist ein Ventil 13 angebracht, welches über eine im Gehäuseteil 1 untergebrachte Steuereinheit 14 steuerbar ist. In bekannter und nicht dargestellter Weise ist im Raum 8, in welchem sich das Piezoelement 3 befindet, ein Niveauregler für die Entkeimungsflüssigkeit untergebracht, mit welchem sich das Niveau der Entkeimungsflüssigkeit regeln lässt, welches das Piezoelement 3 überspült.

Ebenfalls im Gehäuseteil 1 untergebracht ist eine Stromversorgungseinheit 15, welche mit der Steuereinheit 14 verbunden ist und diese mit Strom versorgt. Diese Stromversorgungseinrichtung 15 kann ein Netzteil sein, welcher mit dem Stromnetz verbindbar ist, die Stromversorgungseinrichtung 15 kann aber auch eine Batterie sein, wodurch das Gerät netzunabhängig betrieben werden kann.

Über die Steuereinheit 14 wird das Piezoelement 3 in Schwingung versetzt, das Entkeimungsmittel wird zerstäubt bzw. vernebelt, wobei bei der im wesentlichen konstanten Schwingungsfrequenz von etwa 1,6 MHz abhängig von der Eigenfrequenz des Piezoelementes eine im wesentlichen gleich bleibende Tröpfchengrösse des Entkeimungsmittels erreicht wird.

In der Steuereinheit 14 ist eine Spannungssteuereinheit 17 untergebracht, mit welchem die dem Piezoelement 3 zugeführte Spannung regulierbar ist. Diese Spannungsregelung erfolgt vorzugsweise in einem Bereich zwischen 2 bis 40 Watt. Mit dieser Spannungsregelung lässt sich die Schwingungsamplitude des in Schwingung versetzten Piezoelementes 3 vergrössern bzw. verkleinern, wodurch sich die durch das schwingende Piezoelement 3 zerstäubte bzw. vernebelte Menge des Entkeimungsmittels variieren lässt.

Dies kann auch dadurch erreicht werden, dass die Leistung des Piezoelements 3 mit Hilfe einer bekannten Pulsweitenmodulationsschaltung eingestellt wird. Hierbei wird nicht eine Dauerspannung an das Piezoelement 7 gelegt, sondern in einer bestimmten Schwingungsperiode nur während einer bestimmten Zeitdauer. Diese Zeitdauer kann in Schritten, beispielsweise 32 Schritten, von 0 % auf 100 % erhöht werden.

Über den Ventilator 7, der im Kanal 4 untergebracht ist, und der ebenfalls durch die Steuereinheit 14 geregelt antreibbar ist, lässt sich die Geschwindigkeit des durch den Raum 8 geführten Luftstroms regulieren, z.B. zwischen 0,2 bis 3 m/s. Mit der Regulierung des Luftstroms und der Regulierung der erzeugten Tröpfchen kann die Konzentration des Entkeimungsmittels im abgeführten Luftstrom in einfachster Weise reguliert werden.

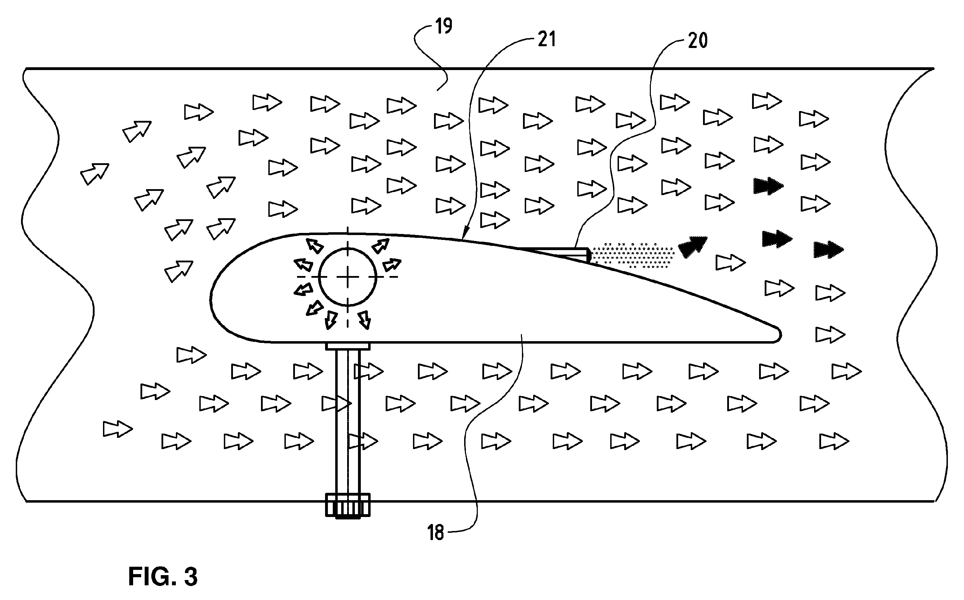

Der aus der Auslassöffnung 6 strömende Luftstrom mit den darin enthaltenden Entkeimungsmitteltröpfchen kann, wie dies in Fig. 3 dargestellt ist, einem Flügelprofil 18 zugeführt werden, das in einem Lüftungskanal 19 angeordnet ist und sich über die Breite des Lüftungskanals 19 erstreckt. Auf der Unterdruckseite 21 des Flügelprofils 18 sind Austrittsöffnungen 20 angebracht. Durch den Unterdruck, der im Bereich dieser Austrittsöffnungen 20 entsteht, wenn die Luft im Lüftungskanal 19 in Pfeilrichtung strömt, wird der Nebel aus dem Flügelprofil 18 durch die Austrittsöffnungen 20 gesaugt und verteilt sich in optimaler Weise in der Luftströmung im Lüftungskanal 19.

Das Flügelprofil 18 kann in bekannter Weise derart im Lüftungskanal 19 untergebracht werden, dass dessen Anstellwinkel zur Strömungsrichtung des im Lüftungskanals 19 strömenden Mediums veränderbar ist. Dadurch lässt sich die Saugwirkung an den Austrittsöffnungen 20 verändern, was sich auf die ausströmende Menge des Entkeimungsmittels in den Lüftungskanal 19 auswirken kann.

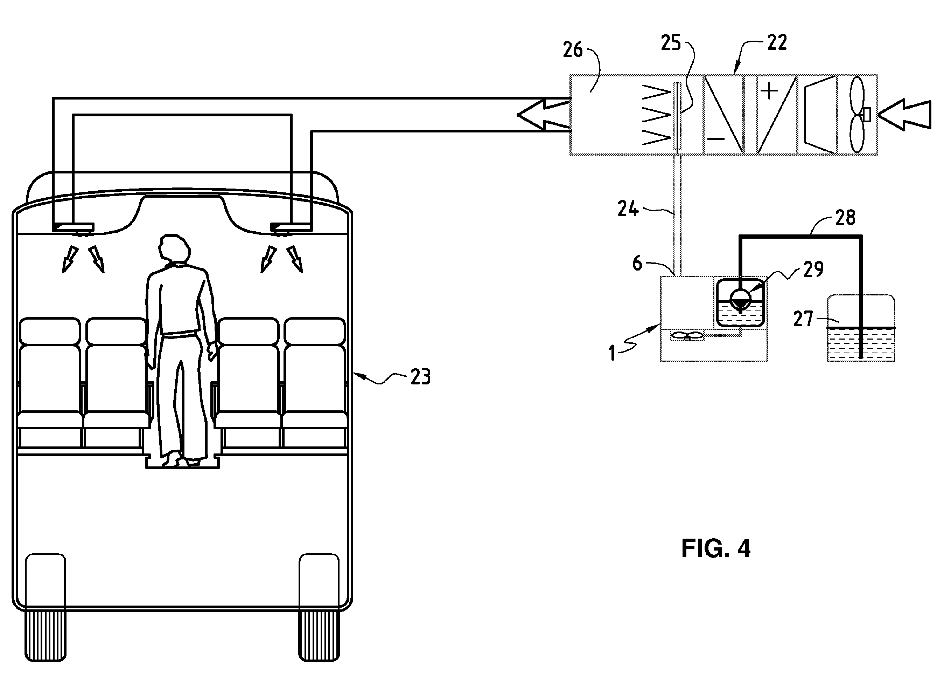

Aus Fig. 4 ist eine Einsatzmöglichkeit eines erfindungsgemässen Gerätes ersichtlich. Dieses Gerät wird in eine bestehende Belüftungseinrichtung 22 für einen Bus 23 eingesetzt. Die Auslassöffnung 6 am Gehäuseteil 1 des erfindungsgemässen Gerätes ist an eine Leitung 24 angekoppelt, die mit einer Lanze 25 verbunden ist, die in den Lüftungskanal 26 oder in das Lüftungsgerät der Belüftungseinrichtung 22 hineinragt. Diese Lanze 25 ist in bekannter Weise mit Öffnungen ausgestattet, wodurch ermöglicht wird, das Entkeimungsmittel in der zerstäubten bzw. vernebelten Form in den Lüftungsstrom im Lüftungskanal 26 schön verteilt einzubringen, welcher Lüftungsstrom dann in den Innenraum des Busses 23 geführt wird. Wie aus dieser Ausgestaltung ersichtlich ist, ist am erfindungsgemässen Gerät ein äusserer Behälter 27 vorgesehen, der über eine Verbindungsleitung 28 mit dem auf dem Gehäuseteil 1 aufgesteckten Pumpenmodul 29 verbunden ist.

Durch diese hier dargestellte Anwendungsmöglichkeit kann die in einem Bus zugeführte Zuluft entkeimt werden. Hierbei lässt sich praktisch jedes gewünschte Entkeimungsmittel einsetzen.

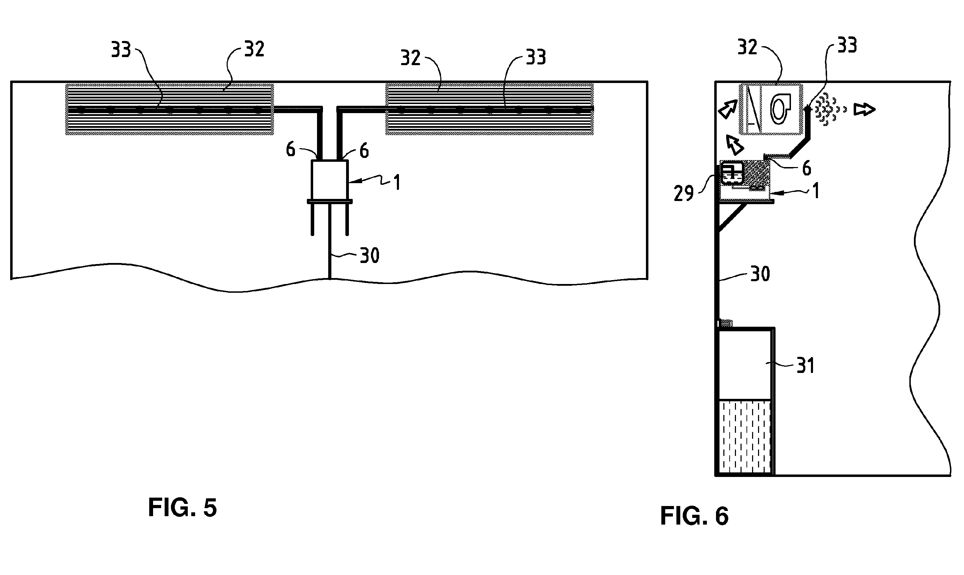

Aus den Fig. 5 und 6 ist der Einsatz eines erfindungsgemässen Gerätes in einem Kühlraum ersichtlich. Hierbei ist der Gehäuseteil 1 des erfindungsgemässen Gerätes wiederum mit einer Leitung 30 mit einem aussenstehenden Behälter 31 verbunden. Hierzu ist auf den Gehäuseteil 1 wiederum ein Pumpenmodul 29 aufgesetzt. Im Bereich des Austritts der Kühlluft aus dem Kühlaggregat 32 ist wiederum eine Lanze 33 angeordnet, die mit der Auslassöffnung 6 am Gehäuseteil des Gerätes verbunden ist, und über welche das Entkeimungsmittel in die Kühlluft ausgeblasen wird und im Kühlraum verteilt wird. Hierbei kann ein Mittel verwendet werden, das beispielsweise den Geruch unterbindet, der durch die im Kühlraum gelagerten Produkte entsteht.

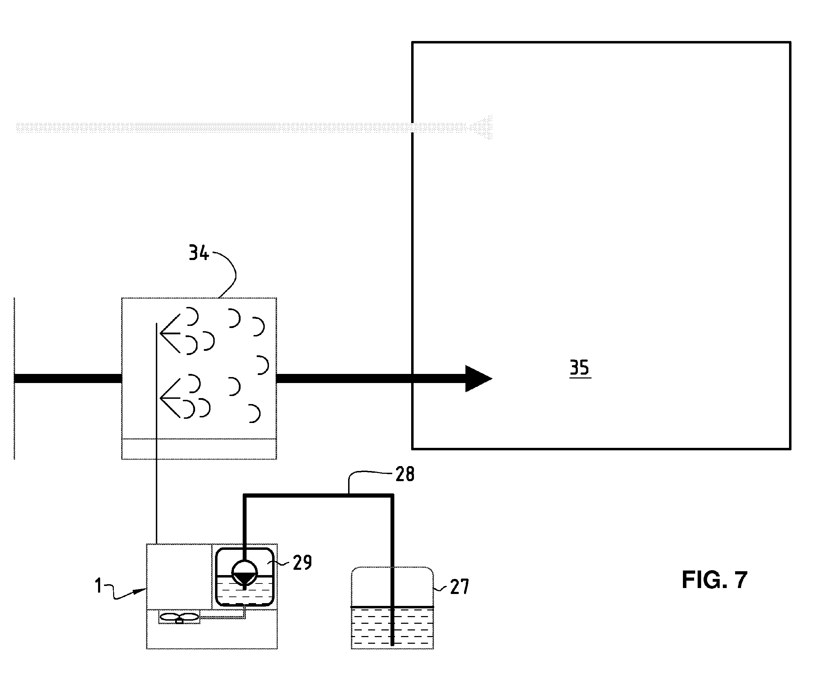

Fig. 7 zeigt den Einsatz eines erfindungsgemässen Gerätes zum Einbringen des Entkeimungsmittels in einer Klimaanlage 34 für ein Gebäude oder Raum 35.

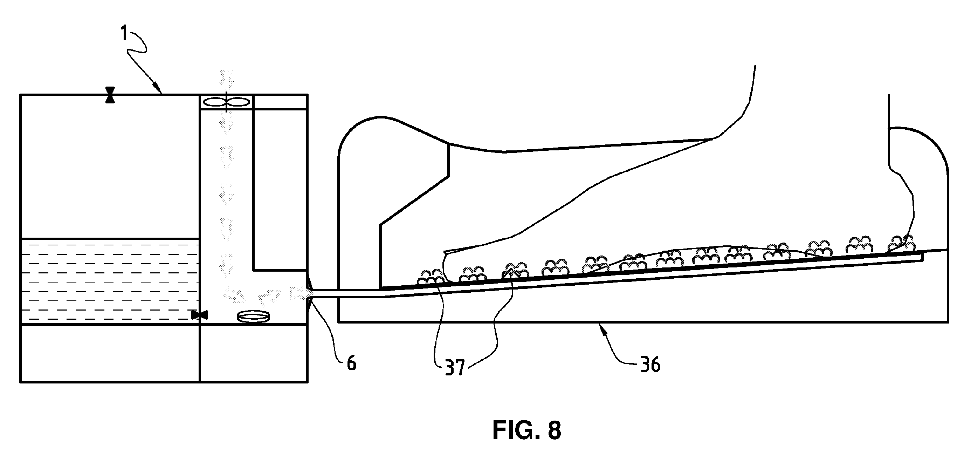

Fig. 8 zeigt eine weitere Anwendung eines erfindungsgemässen Gerätes. Hierbei ist die Auslassöffnung 6, die am Gehäuseteil 1 des Gerätes angebracht ist, und aus welcher das zerstäubte bzw. vernebelte Entkeimungsmittel austritt, über eine Verbindungsleitung mit einem Fussmassagegerät 36 verbunden. Dieses Fussmassagegerät 36 ist im Bereich, wo der Fuss hingestellt wird, mit Austrittdüsen 37 versehen, durch welche das vernebelte bzw. zerstäubte Mittel austreten kann und den Fussunterseitenbereich beaufschlagen kann. Hierbei kann ein Mittel zur Bekämpfung von Fusspilz eingesetzt werden, diese Anwendungsart hat den Vorteil, dass der Fuss nach der Behandlung mit dem entsprechenden Mittel praktisch trocken bleibt.

Das erfindungsgemässe Gerät kann nicht nur für die vorgängig dargestellten Anwendungsgebiete eingesetzt werden, es sind noch viele weitere Möglichkeiten gegeben. Das Gerät kann sehr klein hergestellt werden, dass es sich sogar eignet, in Hosen- oder Jackentaschen mitgeführt zu werden, es kann so zum Beispiel als Inhaliergerät verwendet werden, das jederzeit einsetzbar ist.

Diese Geräte können optimal geregelt werden, die zu verarbeitende Menge des zu zerstäubenden Mittels kann zwischen 1 bis 400 g pro Stunde betragen, je nach Anwendungsfall. Diese Leistung bezieht sich auf ein Piezoelement, selbstverständlich wäre es auch denkbar, in einem Gerät mehrere Piezoelemente unterzubringen, wodurch die Leistung erhöht werden könnte, falls dies gewünscht ist.

Die bei Betrieb des Gerätes entstehende Wärme kann, wenn dies erforderlich ist, in bekannter Weise abgeführt werden.

Selbstverständlich ist dieses Gerät für jede Art von flüssigem Medium verwendbar, das vernebelt bzw. zerstäubt werden soll, je nach Anwendungsfall. Die Verwendung ist nicht nur auf Entkeimungsmittel beschränkt.

## Patentansprüche

1. Gerät zum Einbringen eines flüssigen Mediums, insbesondere Entkeimungsmittels (16) in ein gasförmiges Medium, umfassend einen Gehäuseteil (1), in welchem eine Vernebelungseinrichtung (2) in Form eines Ultraschall-Zerstäubers mit einem Piezoelement (3), welchem das in flüssiger Form vorliegende, in einem Behälter (9) enthaltene Entkeimungsmittel (16) zur Zerstäubung zuführbar ist, ein Kanal (4) mit einer Einlassöffnung (5) und mindestens einer Auslassöffnung (6), durch welchen das gasförmige Medium am Ultraschall-Zerstäuber vorbeiführbar ist und die zerstäubten Partikel des Entkeimungsmittels (16) mitnimmt, ein im Kanal (4) angeordneter Ventilator (7), eine Stromversorgungseinheit (15) für den Ultraschall-Zerstäuber und den Ventilator (7) und eine Steuereinheit (14) untergebracht sind, **dadurch gekennzeichnet, dass** über die Steuereinheit (14) das Piezoelement (3) des Ultraschall-Zerstäubers mit einer vorgegebenen Frequenz, die im Bereich der Eigenfrequenz des Piezoelementes (3) liegt, in Schwingung versetzbar ist, und dass eine Spannungssteuereinheit (17) angebracht ist, mit welchem die an den Ultraschall-Zerstäuber angelegte Spannung veränderbar ist.

2. Gerät nach Anspruch 1, **dadurch gekennzeichnet, dass** die Veränderung der Spannung durch die Steuereinheit (17) durch Einstellen der Amplitude ausführbar ist.

3. Gerät nach Anspruch 1, **dadurch gekennzeichnet, dass** die Veränderung der Spannung durch die Steuereinheit (17) in Form einer Pulsweitenmodulation ausführbar ist.

4. Gerät nach einem der Ansprüche 1 bis 3, **dadurch gekennzeichnet, dass** die Drehzahl des im Kanal (4) angeordneten Ventilators (7) über die Steuereinheit (14) regelbar ist.

5. Gerät nach einem der Ansprüche 1 bis 4, **dadurch gekennzeichnet, dass** die Stromversorgungseinheit (15) als Modul ausgebildet ist und in das Gerät einsetzbar ist.

6. Gerät nach einem der Ansprüche 1 bis 5, **dadurch gekennzeichnet, dass** der Behälter (9) für die Aufnahme des Entkeimungsmittels auf das Gerät aufsteckbar ist, welche mit entsprechenden Steckverbindungen (10, 11) ausgestattet sind.

7. Gerät nach einem der Ansprüche 1 bis 5, **dadurch gekennzeichnet, dass** der Behälter (27; 31) vom Gerät getrennt aufstellbar ist, dass der Behälter (27; 31) mit einer Leitung (28; 30) versehen ist, welche mit einer entsprechenden, am Gerät angebrachten Steckverbindung kuppelbar ist.

8. Gerät nach Anspruch 7, **dadurch gekennzeichnet, dass** auf die Steckverbindung (11) am Gerät ein Pumpenmodul (29) aufsteckbar ist, das über die Stromversorgungseinrichtung (15) speisbar ist, an welches Pumpenmodul (29) die mit dem Behälter (27; 31) verbundene Leitung (28; 30) anschliessbar ist.

9. Gerät nach einem der Ansprüche 1 bis 8, **dadurch gekennzeichnet, dass** die mindestens eine Auslassöffnung (20) auf der Unterdruckseite (21) eines Flügelprofils (18) angeordnet ist, welches Flügelprofil (18) in einem Lüftungskanal (19) einer Lüftungseinrichtung untergebracht ist.

10. Gerät nach Anspruch 9, **dadurch gekennzeichnet, dass** das Flügelprofil (18) sich mindestens über einen Teil der Breite des Lüftungskanals (19) erstreckt und dieses so befestigt ist, dass der Anstellwinkel des Flügelprofils (18) zur Strömungsrichtung des im Lüftungskanals (19) strömenden Mediums veränderbar ist.

11. Gerät nach Anspruch 9 oder 10, **dadurch gekennzeichnet, dass** mehrere Auslassöffnungen (20) verteilt über die Länge des Flügelprofils (18) angeordnet sind.
